# EUROPEAN PATENT APPLICATION

(11) **EP 4 516 217 A1**
(43) Date of publication of application: **05.03.2025**
(21) Application number: 23306425.2
(22) Date of filing: 28.08.2023
(51) Int. Cl.: A61B 5/11, A61B 5/00, G06T 7/20, G16H 50/20, A61B 5/024, A61B 5/08, A61B 5/103, A61B 5/113

(54) **MEDICAL DEVICE FOR DETECTING A MEDICAL CONDITION**

(71) Applicant: Assistance Publique - Hôpitaux de Paris, 75012 Paris (FR)
(72) Inventor: The designation of the inventor has not yet been filed
(74) Representative: IPAZ

(57) **Abstract**

The invention relates to a medical device (2) for detecting the occurrence of a medical condition in a subject (4), the medical device including a processing unit configured to:
- based on a position of a plurality of landmarks, each landmark being representative of a respective joint of the subject (4), compute a trajectory set associated with the subject (4),
the trajectory set including a trajectory of at least one point of interest, each point of interest being a barycenter of a respective landmark set including at least two landmarks;
determine whether the subject (4) exhibits a medical condition or not, based on the computed trajectory set.

## Description

### Field of the invention

The present invention relates to a medical device for detecting the occurrence of a medical condition in a subject.

The invention further relates to a training method and a computer program.

The invention applies to the non-invasive detection of a medical condition in a subject.

### Background

The first 28 days of life (also referred to as "neonatal period") are the most critical for a child's survival, as the first month of life has the highest mortality rate compared to the remaining periods of a child's first years.

Consequently, new-borns undergo a complete clinical examination in the delivery room, before being transferred to the postpartum department.

This clinical examination includes a neurological evaluation, based on observation, which aims to determine if the new-born has neurological impairments.

This observational examination is essential, as it makes it possible to evaluate cardinal functions of the new-born (namely motricity, reactivity and interaction), and to approach the functioning of the central and peripheral neurological structures of the new-born. Indeed, spontaneous motor activity of the new-born is a reflex motor activity correlated to the age and to the condition of the new-born. This spontaneous motor activity is reproducible and homogeneous from a patient to another, and quite recognisable for a trained health care professional.

Motor analysis in the new-born begins with the general movements assessment (GMA). General movements (GM) are movements that appear around 8-9 weeks of amenorrhea and are observable until 15-20 weeks. They are then gradually replaced by voluntary movements. General movements are characterized by a variable sequence of the head, trunk, arm, and leg movements. The intensity, strength, and velocity of the movements are also variable and related to the patient's condition. The flexion and extension movements of the upper and lower limbs become more complex as rotations are added to the movements. This also applies to changes in the direction of movement.

Since the aforementioned cardinal functions are altered during systemic physio-pathological phenomena for which the neurological attack is only secondary or indirect (for instance: sepsis, metabolic diseases, hypoxic-ischemic encephalopathies, etc.), such neurological evaluation also allows to evaluate the new-born's general health.

The first and main deviation from the normal motor condition (or normal motor pattern) is often called hypotonia. Hypotonia is defined as a global decrease in spontaneous activity resulting in movements that are less frequent, slower, and with a reduced range compared to normal movements. These modifications of the global motor pattern of the new-born can vary in intensity and in presentation, and can strongly suggest a diagnostic orientation if read correctly.

Moreover, this motor activity pattern deviation often appears early in the course of the disease (regardless of the disease). Yet, at such early stages, the underlying disease, if diagnosed immediately, is often reversible thanks treatments that are very effective if they are provided early enough to the infant, thereby preventing sequelae. Such treatments may be symptomatic (such as hypothermia in the case of hypoxic-ischemic encephalopathy), antibiotics (in the event of maternal-foetal infection) or more even genetic (for spinal muscular atrophy, etc.). This makes such observational neurological evaluation all the more important.

However, despite the standardization of observation and data collection, such observational neurological evaluation involves a part of subjectivity based on the experience of the practitioner, thereby potentially compromising the accuracy of the observational neurological evaluation. Furthermore, it is generally very difficult to access specialized practitioners in a decent amount of time, which is prejudicial to the new-born, since the aforementioned diseases can evolve to life threatening conditions in a matter of mere hours.

A purpose of the present invention is to overcome at least one of these drawbacks.

Another purpose of the invention is to provide a solution for a more accurate and reliable examination.

Yet another purpose of the invention is to allow non-invasive early identification of a medical condition or a pathology.

### Summary of the invention

To this end, the present invention is a medical device of the aforementioned type, including a processing unit configured to:
- based on a position of a plurality of landmarks, each landmark being representative of a respective joint of the subject, compute a trajectory set associated with the subject,
   the trajectory set including a trajectory of at least one point of interest, each point of interest being a barycenter of a respective landmark set including at least two landmarks;
- determine whether the subject exhibits a medical condition or not, based on the computed trajectory set.

Indeed, by observing the trajectory of the barycenter of at least two landmarks, each representative of a respective joint of the subject, movement patterns of the subjects can be determined. For instance, in the case where the landmarks belong to the same limb, a general movement pattern of the corresponding limb is determined based on the observation of the trajectory of the associated barycentre.

The inventors have discovered the surprising effect that such trajectory of the barycenter (and the related movement pattern) is an objective and very reliable tool to perform a neurological evaluation of a subject, since neuromuscular disfunctions and musculoskeletal lesions have the particularity of modifying the amplitude and direction of barycenter displacement. This enhanced neurological and orthopedic evaluation consequently allows to identify a medical condition that may be the cause of an abnormal movement pattern of the subject.

Thanks to the invention, early warning signs of a medical condition can be detected, which can help addressing the patient to the right practitioner with shorter delay. The invention also enables longitudinal assessment and monitoring of limb mobility, optimizing diagnosis, treatment and follow-up of patients throughout their lives.

The device may be integrated as a "software as medical device" or a personal device such as a smartphone, a tablet, a Smartwatch, a computer, any wearable electronic device, etc.

The device according to the invention may execute one or several applications to carry out the method according to the invention.

The device according to the invention may be loaded with, and configured to execute, the computer program according to the invention.

According to other advantageous aspects of the invention, the device includes one or several of the following features, taken alone or in any technically possible combination:

for each landmark, the processing unit is configured to perform smoothing of a corresponding position over time before computing the trajectory set, preferably by implementing a Savitzky-Golay filter;

before computing the trajectory set, the processing unit is configured to, for at least two frames of a video showing at least part of the subject's body:
- detect each corresponding landmark; and
- determine the position of each detected landmark;

The medical device according to claim 3, wherein the processing unit is further configured to perform, before detection of the landmarks, a preprocessing including:
- a contrast modification comprising, for each frame of the video:
   - modifying the contrast of the frame in a stepwise manner, within a predetermined contrast range, to generate a set of modified frames;
   - for each modified frame of the set of modified frames, performing a preliminary detection of the landmarks;
   - retaining the modified frame for which a confidence in the preliminary detection is the highest and, preferably, is above a predetermined threshold; and/or
- performing a background rejection including:
   - segmenting the subject's body to discriminate a background;
   - removing the background from the frame;

the processing unit is further configured to perform a processing of the video to remove a shaking noise representative of a movement of a camera outputting the video relative to the subject;

the medical device further includes a camera connected to the processing unit, the camera being configured to acquire the video of the subject and to output the acquired video to the processing unit;

processing unit is configured to track a distance between two landmarks associated with two consecutive joints of a same limb,
the processing unit being further configured to:
- determine a corresponding reference length representative of a maximum distance between the two landmarks; and
- compute a position along a depth direction of each landmark based on the determined reference length;

the processing unit is further configured to determine whether the subject exhibits a medical condition or not based on the computed trajectory set and on at least one biomarker,
each biomarker being representative of at least one of: an age of the subject, a mood of the subject, a gaze of the subject, sounds emitted by the subject and/or a head orientation of the subject;

the processing unit is further configured to determine each biomarker based on a video showing at least part of the subject's body;

the processing unit is further configured to identify, based on the computed trajectory set, whether the subject exhibits at least one of:
- a kinematic asymmetry between limbs;
- an abnormal activity with respect to a healthy reference subject;
- an activity modification with respect to a previous state of the subject;
- a difference between a subject locomotor pattern with respect to a predetermined ideal locomotor pattern;

the processing unit is further configured to monitor an evolution of the medical condition over time, based on the computed trajectory set;

for each landmark set, the respective point of interest is the corresponding centroid;

the processing unit is configured to implement an artificial intelligence model previously trained to determine whether a trajectory set associated with a subject is indicative of either an abnormal movement pattern, representative of the occurrence of a medical condition in the subject, or a normal movement pattern.

The invention further relates to a configuration method for configuring an artificial intelligence model to detect the occurrence of a medical condition in a subject, the configuration method including training the artificial intelligence model based on training data comprising:
- at least one trajectory set, each associated with a respective subject and including a trajectory of at least one barycenter, each barycenter being a barycenter of at least two landmarks, each landmark being representative of a respective joint of the subject;
- for each trajectory set, a label indicating whether the subject exhibits a medical condition or not.

According to another aspect of the same invention, it is proposed a computer program comprising instructions, which when executed by a computer, cause the computer to carry out the following steps:
- based on a position of a plurality of landmarks, each landmark being representative of a respective joint of a subject, computing a trajectory set associated with the subject,
   the trajectory set including a trajectory of at least one point of interest, each point of interest being a barycenter of a respective landmark set including at least two landmarks;
- determining whether the subject exhibits a medical condition or not, based on the computed trajectory set.

The computer program may be in any programming language such as C, C++, JAVA, Python, etc.

The computer program may be in machine language.

The computer program may be stored, in a non-transient memory, such as a USB stick, a flash memory, a hard-disc, a processor, a programmable electronic chop, etc.

The computer program may be stored in a computerized device such as a smartphone, a tablet, a computer, a server, etc.

### Brief description of the figures

Other advantages and characteristics will become apparent on examination of the detailed description of an embodiment which is in no way limitative, and the attached figures, where:
Figure 1 is a schematic representation of a medical device according to the invention;
Figure 2 is a flowchart of a detection method performed by the medical device of figure 1;
Figure 3 is a schematic representation of landmarks used by the medical device of figure 1; and
Figure 4 is a graph representing a trajectory set computed from a video of a subject.

It is well understood that the embodiments that will be described below are in no way limitative. In particular, it is possible to imagine variants of the invention comprising only a selection of the characteristics described hereinafter, in isolation from the other characteristics described, if this selection of characteristics is sufficient to confer a technical advantage or to differentiate the invention with respect to the state of the prior art. Such a selection comprises at least one, preferably functional, characteristic without structural details, or with only a part of the structural details if this part alone is sufficient to confer a technical advantage or to differentiate the invention with respect to the prior art.

In the figures, elements common to several figures retain the same reference.

### Detailed description

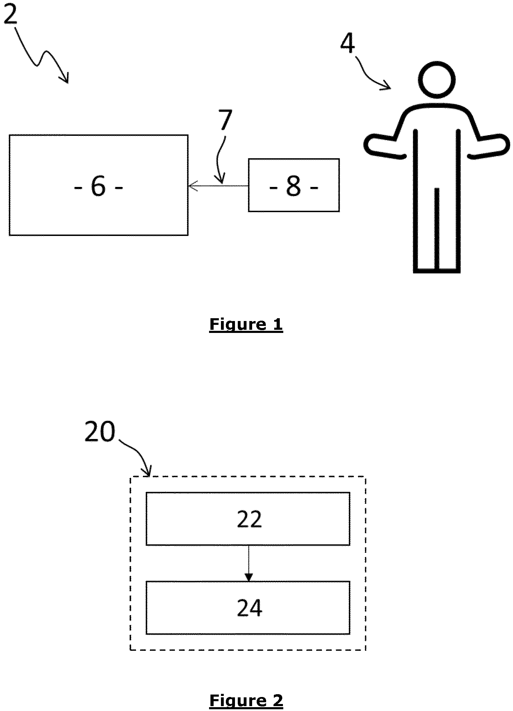

A medical device 2 according to the invention is shown on figure 1.

The medical device 2 is configured to detect the occurrence of at least one medical condition in a subject 4, based on movements of said subject 4.

Preferably, the medical device 2 is configured to detect whether the subject 4 exhibits at least one of the following musculoskeletal and/or neurological dysfunctions:
- kinematic asymmetry between limbs (amplitude, angulation, velocity, acceleration, etc.);
- abnormal activity with respect to a healthy reference subject, such as lack of mobility or excessive mobility of body parts (head, trunk, limbs, etc.);
- activity modification with respect to a previous state of the same subject (loss of motion capacities, modification after treatment, modification during time and growth, modification after training or rehabilitation);
- difference between subject activity and motion with respect to an ideal gesture or locomotor pattern.

More precisely, the medical device 2 includes a processing unit 6 configured to detect the occurrence of each medical condition based on a video 7 of the subject 4.

Preferably, the medical device further includes a camera 8 connected to the processing unit 6. In this case, the camera 8 is configured to acquire the video 7 of the subject 4, and to output the acquired video 7 to the processing unit 6.

The camera 8 may be, for example, a fixed camera, such as a webcam configured to output the video 7 as a video stream (e.g., a continuous video stream) to the processing unit 6. This is, for instance, the preferred option for on-site monitoring of patients in a health facility such as a hospital.

Alternatively, the camera 8 may be a handheld device. In this case, the camera 8 (*e.g*., such as a smartphone) may be distinct from medical device 2, and may be configured to upload the acquired video 7 to the processing unit 6. This embodiment is advantageous, as it allows off-site monitoring of patients, *i.e*., remote monitoring of patients that are not present on the premises of a health facility.

### Processing unit 6

A detailed description of the processing unit 6 will now be provided with reference to figures 2 to 5.

To detect the occurrence of a medical condition in the patient 4, the processing unit 6 is configured to implement a detection method 20 (figure 2).

The detection method 20 includes a trajectory computation step 22 and a prediction step 24.

### Trajectory computation step 22

Preferably, for each frame of the video 7, the processing unit 6 is configured to detect, during the trajectory computation step 22, the joints of the subject 4, and to associate a landmark 30 with each of the detected joints.

For instance, to detect the joints of the subject 4, thereby determining each landmark 30, the processing unit 6 is configured to implement the OpenPose library created by Ginés Hidalgo et al.

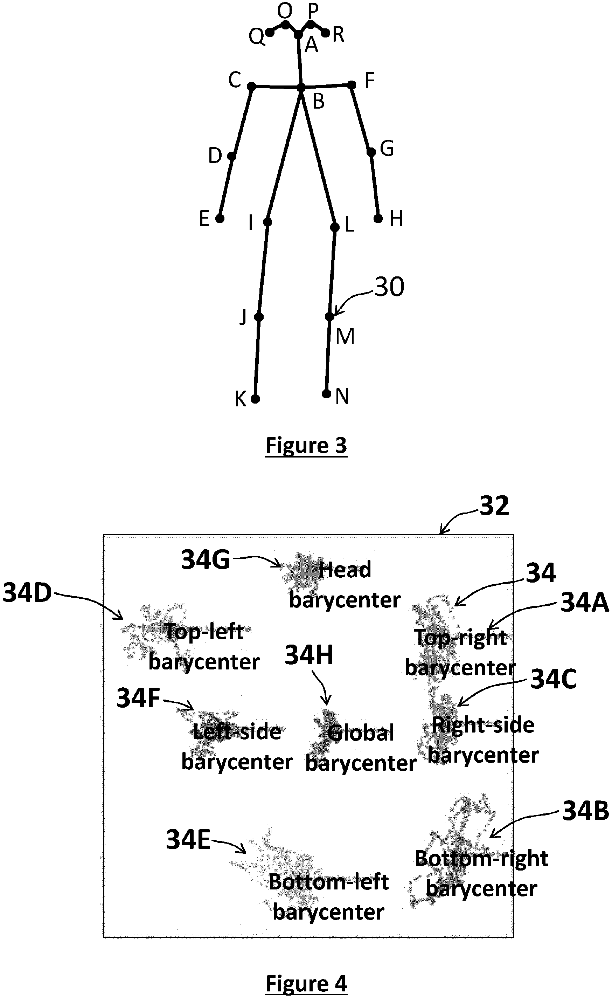

An example of landmarks 30 detected in a frame of a video of a subject 4 is shown on figure 3. More precisely, each node of the graph represented on figure 3 is a landmark corresponding to a respective joint.

In this example:
- landmark B corresponds to the neck;
- landmark C corresponds to the right shoulder;
- landmark D corresponds to the right elbow;
- landmark E corresponds to the right wrist;
- landmark F corresponds to the left shoulder;
- landmark G corresponds to the left elbow;
- landmark H corresponds to the left wrist;
- landmark I corresponds to the right hip joint;
- landmark J corresponds to the right knee;
- landmark K corresponds to the right ankle;
- landmark L corresponds to the left hip joint;
- landmark M corresponds to the left knee; and
- landmark N corresponds to the left ankle of the subject 4.

Moreover, nodes A, O, P, Q and R correspond respectively to the nose, the right eye, the left eye, the right ear and the left ear of the subject 4.

Advantageously, to enhance the detection of the landmarks, the processing unit 6 is configured to perform image processing on the video 7.

For instance, the processing unit 6 is configured to perform a processing of the video 7 to remove a shaking noise representative of (*i.e.,* caused by) a movement of the camera 8. This is especially advantageous in the case where the camera 8 is a handheld device.

Alternatively, or in addition, for each frame of the video 7, the processing unit 6 is configured to perform a background rejection.

More precisely, in order to perform the background rejection, the processing unit 6 is configured to:
- segment the subject's body in the frame to discriminate a background;
- remove the background from the frame.

By "removing the background", it is meant, in the context of the present invention, affecting a predetermined value to each pixel identified as being part of the background. This enhances the ability of discriminating the subject from the remaining elements in the image.

Alternatively, or in addition, for each frame of the video 7, the processing unit 6 is configured to perform a contrast modification of said frame.

More precisely, for each frame of the video, in order to perform the contrast modification, the processing unit 6 is configured to:
- modify the contrast of the frame in a stepwise manner, within a predetermined contrast range, to generate a set of modified frames;
- perform, for each modified frame of the set of modified frames, a preliminary detection of the landmarks;
- retain the modified frame for which a confidence in the preliminary detection is the highest and, preferably, is above a predetermined threshold.

Alternatively, for each frame of the video 7, each landmark is detected by an image processing unit (not shown) distinct from the medical device 2.

Moreover, the processing unit 6 is preferably configured to, for at least two frames of the video 7, determine the position of each detected landmark 30, *i.e.,* its position in a predetermined coordinate system.

Advantageously, the processing unit 6 also is configured to estimate a distance between each landmark 30 and the camera 8, that is to say a position along a depth direction of each landmark 30.

To do so, the processing unit 6 is configured to track, in the video 7, a distance between two landmarks 30 associated with two consecutive joints of a same limb.

The processing unit 6 is further configured to determine a corresponding reference length representative of a maximum distance between said two landmarks 30. This reference length is obtained when both landmarks (i.e., both joints) define a line that is orthogonal to a line of sight of the camera 8.

Finally, the processing unit 6 is configured to compute a position along a depth direction of each landmark 30 based on the determined reference length.

Moreover, the processing unit 6 is configured to compute, during the trajectory computation step 22, a trajectory set 32 associated with the subject 4, based on a position of a plurality of landmarks 30. An exemplary trajectory set 32 is shown on figure 4. In this figure, the trajectory set 32 is shown in a plane of a sensor of camera 8.

The trajectory set 32 includes at least one trajectory 34. Each trajectory 34 is a trajectory of a corresponding point of interest 36, defined as a barycentre of a respective landmark set comprising at least two landmarks 30. More precisely, for each point of interest 36, the corresponding trajectory includes the position of said point of interest 36, in the predetermined coordinate system, determined based on each frame of the video 7.

Preferably, the point of interest 36 is the centroid of the respective landmark set.

In the example of figure 4, the trajectory set 32 includes eight trajectories 34. More precisely:
- trajectory 34A corresponds to the trajectory of the centroid of landmarks F, G and H;
- trajectory 34B corresponds to the trajectory of the centroid of landmarks L, M and N;
- trajectory 34C corresponds to the trajectory of the centroid of landmarks F, G, H, L, M and N;
- trajectory 34D corresponds to the trajectory of the centroid of landmarks C, D and E;
- trajectory 34E corresponds to the trajectory of the centroid of landmarks I, J and K
- trajectory 34F corresponds to the trajectory of the centroid of landmarks C, D, E, I, J and K;
- trajectory 34G corresponds to the trajectory of the centroid of landmarks A and B, as well as nodes O, P, Q and R;
- trajectory 34H corresponds to the trajectory of the centroid of landmarks all the nodes A to R.

Preferably, the joints corresponding to a given point of interest 36 (i.e., the landmarks 30 of the respective landmark set) have been previously defined by an operator during a preliminary configuration step of the medical device 2.

Advantageously, for each landmark 30, the processing unit 6 is configured to perform a smoothing of a corresponding position over time before computing the trajectory set 32. Thanks to this smoothing, the noise and jitter between frames, caused by the fact that the detection of the landmarks 30 is performed in each frame independently from the other frames, are reduced, or even removed.

In this case, the processing unit 6 is preferably configured to perform such smoothing by implementing a Savitzky-Golay filter. Such filter, which operates as a polynomial low-pass filter, is effective in reducing noise and undesirable fluctuations in estimated joint trajectories. Savitzky-Golay filtering is also advantageous due to the fact that it is very efficient and easy to implement. As an example, the inventors have set the parameters to a polynomial degree of 3 and a window of 21 to the motion of each point of each video. This allowed to remove this noise without affecting diagnosis.

### Prediction step 24

As stated previously, the processing unit 6 is also configured to determine, during the prediction step 24, whether the subject exhibits a medical condition or not, based on the computed trajectory set.

Preferably, to perform such determination, the processing unit 6 is configured to implement an artificial intelligence model previously trained to determine whether a trajectory set 32 associated with the subject is indicative of either an abnormal movement pattern, representative of the occurrence of a medical condition in the subject, or a normal movement pattern, representative of a healthy subject.

For instance, in this case, the artificial intelligence model has been previously trained based on training data comprising:
- at least one trajectory set, each associated with a respective test subject and including a trajectory of at least one barycenter, each barycenter being a barycenter of at least two landmarks, each landmark being representative of a respective joint of the test subject;
- for each trajectory set, a label indicating whether the test subject exhibits a medical condition or not.

In this case, for each trajectory set, the label preferably further indicates which medical condition the test subject exhibits, if any.

Advantageously, each medical condition to be detected is associated with a respective artificial intelligence model specifically trained to detect, based on a trajectory set 32, the occurrence of said medical condition in the subject 4.

Advantageously, the processing unit 6 is further configured to determine whether the subject exhibits a medical condition or not based on:
- the computed trajectory set; and
- at least one biomarker.

For instance, each biomarker is representative of at least one of: an age of the subject, a mood of the subject, a gaze of the subject, sounds emitted by the subject and/or a head orientation of the subject.

In this case, the processing unit 6 is preferably configured to determine at least one biomarker based on the video 7.

Preferably, the processing unit 6 is also configured to determine, during the prediction step 24, at least one vital sign of the subject, preferably a respiratory rate and/or a heart rate.

For instance, the processing unit 6 is also configured to determine heart rate based on color variation analysis and/or photoplethysmography image processing.

Color variation analysis is based on the fact that skin color changes slightly according to the blood flow induced by the heartbeat. Consequently, by measuring these color variations from the video, the processing unit 6 is able to determine the heart rate. For instance, to perform color variation analysis, the processing unit 6 is configured to implement an analysis of the chrominance component (such as the green channel), or to use pattern detection methods.

For instance, the processing unit 6 is also configured to determine respiratory rate based on an analysis of thoracic movements and/or contour variation analysis.

More precisely, analysis of thoracic movements relates to estimating breathing from a video based on an analysis of movements of the torso or abdomen. Using tracking or object detection techniques, the processing unit 6 can extract regions of interest corresponding to respiratory movements and calculate respiratory rate from this information.

Alternatively, or in addition, the processing unit 6 is configured to detect variations in the contour of the thoracic or abdominal region caused by breathing (for instance using contour detection or tracking algorithms) and to determine the respiratory rate based on said variations.

Thanks to the invention, there is no need to use fiducial markers placed on the body of the subject 4 to detect the occurrence of a medical condition. Consequently, the medical device 2 is not intrusive, time-saving, affordable and reliable.

Another drawback of the aforementioned markers is that their weight influenced the newborn's motor skills, thereby biasing their movements. Thanks to the invention, this problem is overcome.

### Operation

Operation of the medical device 2 will now be disclosed.

Preferably, for each frame of the video 7, the processing unit 6 detects, during the trajectory computation step 22, the joints of the subject 4, and associates a landmark 30 with each of the detected joints.

Advantageously, to enhance the detection of the landmarks, the processing unit 6 performs image processing on the video 7.

Moreover, the processing unit 6 preferably determines, for at least two frames of the video 7, the position of each detected landmark 30.

Advantageously, the processing unit 6 also estimates a position along a depth direction of each landmark 30.

Advantageously, for each landmark 30, the processing unit 6 performs a smoothing of a corresponding position over time.

Furthermore, the processing unit 6 computes, during the trajectory computation step 22, a trajectory set 32 associated with the subject 4, based on a position of a plurality of landmarks 30 among the detected landmarks 30.

Then, the processing unit 6 determines, during the prediction step 24, whether the subject exhibits a medical condition or not, based on the computed trajectory set.

Advantageously, the processing unit 6 further determines whether the subject exhibits a medical condition or not based on:
- the computed trajectory set; and
- at least one biomarker.

Preferably, the processing unit 6 also determines, during the prediction step 24, and based on the trajectory set 32, at least one vital sign of the subject, preferably a respiratory rate and/or a heart rate.

## Claims

1. A medical device (2) for detecting the occurrence of a medical condition in a subject (4), the medical device including a processing unit configured to:
- based on a position of a plurality of landmarks (30), each landmark (30) being representative of a respective joint of the subject (4), compute a trajectory set (32) associated with the subject (4),
the trajectory set (32) including a trajectory (34) of at least one point of interest,
each point of interest being a barycenter of a respective landmark set including at least two landmarks (30);
- determine whether the subject (4) exhibits a medical condition or not, based on the computed trajectory set (32).

2. The medical device (2) according to claim 1, wherein, for each landmark, the processing unit is configured to perform smoothing of a corresponding position over time before computing the trajectory set (32), preferably by implementing a Savitzky-Golay filter.

3. The medical device (2) according to claim 1 or 2, wherein before computing the trajectory set (32), the processing unit is configured to, for at least two frames of a video showing at least part of the subject's body:
- detect each corresponding landmark; and
- determine the position of each detected landmark.

4. The medical device (2) according to claim 3, wherein the processing unit is further configured to perform, before detection of the landmarks (30), a preprocessing including:
- a contrast modification comprising, for each frame of the video:
• modifying the contrast of the frame in a stepwise manner, within a predetermined contrast range, to generate a set of modified frames;
• for each modified frame of the set of modified frames, performing a preliminary detection of the landmarks (30);
• retaining the modified frame for which a confidence in the preliminary detection is the highest and, preferably, is above a predetermined threshold; and/or
- performing a background rejection including:
• segmenting the subject's body to discriminate a background;
• removing the background from the frame.

5. The medical device (2) according to claim 3 or 4, wherein the processing unit is further configured to perform a processing of the video to remove a shaking noise representative of a movement of a camera outputting the video relative to the subject (4).

6. The medical device (2) according to any one of claims 3 to 5, further including a camera connected to the processing unit, the camera being configured to acquire the video of the subject (4) and to output the acquired video to the processing unit.

7. The medical device (2) according to any one of claims 1 to 6, wherein the processing unit is configured to track a distance between two landmarks (30) associated with two consecutive joints of a same limb,
the processing unit being further configured to:
- determine a corresponding reference length representative of a maximum distance between the two landmarks (30); and
- compute a position along a depth direction of each landmark based on the determined reference length.

8. The medical device (2) according to any one of claims 1 to 7, wherein the processing unit is further configured to determine whether the subject (4) exhibits a medical condition or not based on the computed trajectory set (32) and on at least one biomarker,
each biomarker being representative of at least one of: an age of the subject (4), a mood of the subject (4), a gaze of the subject (4), sounds emitted by the subject (4) and/or a head orientation of the subject (4).

9. The medical device (2) according claim 8, wherein the processing unit is further configured to determine each biomarker based on a video showing at least part of the subject's body.

10. The medical device (2) according to any one of claims 1 to 11, wherein the processing unit is further configured to identify, based on the computed trajectory set (32), whether the subject (4) exhibits at least one of:
- a kinematic asymmetry between limbs;
- an abnormal activity with respect to a healthy reference subject;
- an activity modification with respect to a previous state of the subject;
- a difference between a subject locomotor pattern with respect to a predetermined ideal locomotor pattern.

11. The medical device (2) according to any one of claims 1 to 10, wherein the processing unit is further configured to monitor an evolution of the medical condition over time, based on the computed trajectory set (32).

12. The medical device (2) according to any one of claims 1 to 11, wherein, for each landmark set, the respective point of interest is the corresponding centroid.

13. The medical device (2) according to any one of claims 1 to 12, wherein the processing unit is configured to implement an artificial intelligence model previously trained to determine whether a trajectory set (32) associated with a subject is indicative of either an abnormal movement pattern, representative of the occurrence of a medical condition in the subject (4), or a normal movement pattern.

14. A configuration method for configuring an artificial intelligence model to detect the occurrence of a medical condition in a subject (4), the configuration method including training the artificial intelligence model based on training data comprising:
- at least one trajectory set (32), each associated with a respective subject and including a trajectory of at least one barycenter, each barycenter being a barycenter of at least two landmarks (30), each landmark being representative of a respective joint of the subject;
- for each trajectory set (32), a label indicating whether the subject exhibits a medical condition or not.

15. Computer program comprising instructions, which when executed by a computer, cause the computer to carry out the following steps:
- based on a position of a plurality of landmarks (30), each landmark being representative of a respective joint of a subject (4), computing a trajectory set (32) associated with the subject (4),
the trajectory set (32) including a trajectory of at least one point of interest,
each point of interest being a barycenter of a respective landmark set including at least two landmarks (30);
- determining whether the subject (4) exhibits a medical condition or not, based on the computed trajectory set (32).
